# EUROPEAN PATENT APPLICATION

(11) **EP 4 338 798 A1**
(43) Date of publication of application: **20.03.2024**
(21) Application number: 23197564.0
(22) Date of filing: 15.09.2023
(51) Int. Cl.: A61P 27/06, A61K 36/16, A61K 45/06, A61K 9/00, A61K 31/352

(54) **PHARMACEUTICAL PREPARATION CONTAINING GENISTEIN FOR USE IN THE PREVENTION OR TREATMENT OF GLAUCOMA AND/OR OCULAR HYPERTENSION**

(30) Priority: 16.09.2022 PL 44230122
(71) Applicant: Wasilewicz, Robert Henryk, 61-048 Poznan (PL); Kaluzny, Marcin, 61-052 Poznan (PL)
(72) Inventor: Wasilewicz, Robert Henryk, 61-048 Poznan (PL); Kaluzny, Marcin, 61-052 Poznan (PL)
(74) Representative: Wojasinska, Ewa

(57) **Abstract**

The subject matter of the invention is a pharmaceutical formulation for use in prevention or treatment of ocular hypertension and/or glaucoma in a patient, comprising a pharmaceutically acceptable carrier, diluent, excipient and/or adjuvant, characterized in that it comprises genistein in an amount of 10 to 15 % by weight based on the total weight of the pharmaceutical formulation, which also comes in the form of a capsule with a HPMC shell.

## Description

### Field of the invention

The subject matter of the invention is a pharmaceutical formulation comprising genistein for use in prevention or treatment of glaucoma and/or ocular hypertension. In particular, the invention includes a pharmaceutical formulation comprising genistein, obtained particularly from the Japanese pagoda tree fruit, for use in prevention or treatment of glaucoma and/or ocular hypertension to protect trabecular meshwork cells, lamina cribrosa of the sclera cells and retinal ganglion cells.

### Background of the invention

The invention relates to the field of medical sciences - ophthalmology, and glaucoma and/or ocular hypertension in particular.

Glaucoma is an eye disease that damages the optic nerve and leads to gradual loss of vision. The key to controlling the disease is to strive for the protection of retinal ganglion cells that form the optic nerve. Their gradual disappearance, along with the disease progress, leads to irreversible visual field loss. The main risk factor for the development and progression of optic nerve damage in glaucoma is increased pressure inside the eyeball - the intraocular pressure (IOP). Ocular hypertension is a disease characterized by elevated IOP without evidence of optic nerve damage. People diagnosed with ocular hypertension are in a group at risk of developing glaucoma.

Currently, glaucoma treatment involves reducing intraocular pressure by using drugs that facilitate the outflow of an aqueous humor from the eyeball and/or reducing its production. Mainly topical drugs are used in the form of eye drops, sometimes oral drugs are administered. Other methods of decreasing intraocular pressure are laser therapy and surgical procedures.

In recent years, research teams in Poland and around the world have been focusing on various aspects of the biological activity of genistein, looking for new medical applications of this isoflavone. This is confirmed by numerous scientific articles in nature and medicine-oriented journals. There are reports of the use of genistein in the field of ophthalmology.

From the patent EP 2129373 B1 the use of genistein for preparation of a composition for treatment or prevention of dry eye syndrome or lacrimal keratoconjunctivitis in humans or animals is known. The specification discloses a composition for systemic administration, preferably for oral use. This agent is in the form of a pharmaceutical dose or is a food or feed supplement, etc. and is used in the field of ophthalmology, however, it is not intended for use in prevention and treatment of glaucoma.

From the patent application US20080181972 A1 a nutritional or dietary composition comprising genistein in the composition in an amount ranging from 10 to 200 mg (paragraph 103; claims 3, 5, claims 15 to 17) for daily ingestion is known. The application also discloses a method of maintaining, enhancing, ameliorating or promoting eye health in a patient including administering to said patient a nutritional or dietary composition comprising genistein or a pharmaceutical equivalent thereof (claims 18 to 19), wherein the said patient suffers from an eye disease (claim 25) or is at risk of developing it, and glaucoma is mentioned among eye diseases (claim 26).

Patent US6399655 B1 discloses the use of genistein in the field of ophthalmology for preventive treatment of the eye disease - cataract. In claim 21 it is also indicated that the cataract may result from a systemic disorder, a dermatological disorder, a central nervous system disorder or a local eye disease, being, among others, glaucoma.

The publication of the application DE102014203152 A1 discloses a micro- or nanoemulsion for ophthalmic use comprising among others genistein in the composition (claim 5). These emulsions are intended for use in treatment or prevention of glaucoma.

The patent application US20160367497 A1 discloses a method of promoting axonal protection in a mammal in need of treatment for optic neuropathy including administering to the mammal an agent in an effective amount to promote axonal protection, where the neuropathy is, among others, glaucoma (claim 6), and the agent is among others genistein isoflavone (claim 15).

The publication of the application US20200009078 A1 discloses an oral composition comprising genistein in its composition (claim 4), which composition may be intended for treatment of glaucoma (claim 13).

However, there is an ongoing need for new solutions comprising pharmaceutical and therapeutic formulations, compositions or agents for non-surgical treatment of glaucoma and/or ocular hypertension.

There is a known glaucoma therapy-enhancing "GlauCaps" formulation on the market comprising genistein from Japanese pagoda tree fruit (98% extract) (the offer from an online pharmacy of the "GlauCaps 30 kaps" formulation; the webpage archived on 19.05.2019 http://web.archive.org/web/20190519210554/http://aptekawaw.pl/-p-87345.html;
a website with the information about the "GlauCaps" formulation: http://web.archive.org/web/20210621161642/http://www.glaucaps.com/glaucaps-c-310.html webpage archived on 21.06.2021, slides;
and a webpage concerning the "GlauCaps" formulation: https://web.archive.org/web/20210621164722/http://www.glaucaps.com/?osCsid=b1b6c2c 46c56cfc7e62490296f3ba9c5 archived on 21.06.2021). These publications do not comprise data on the quantitative composition of the formulation used.

The above-mentioned known "GlauCaps" formulation commercially available from OphthaPharm sp. z o.o., for the purposes of this patent specification is hereinafter referred to as "GlauCaps V1" "V1 formulation" or "V1". It comprises in the composition a soybean oil, a green tea leaf extract comprising 50 % of ECGC, pork gelatin, a humectant: glycerol, a gingko biloba leaf extract comprising 24 % of flavonoid glycosides and 6 % of terpene lactones (Egb761), a Japanese pagoda tree fruit extract comprising 98 % of genistein, an anti-caking substance: yellow beeswax, zinc oxide, selenomethionine, a fully hydrogenated coconut oil, an emulsifier: lecithins (soybean based), dyes: iron oxides and hydroxides, titanium dioxide.

However, regarding the GlauCaps V1 formulation mentioned above, there is no data on its quantitative composition, including the amount of genistein in the formulation. Quantitative composition of the GlauCaps V1 formulation is characterized only (e.g. in the offer from an online pharmacy http://web.archive.org/web/20220526085745/https://gemini.pl/glaucaps-30-kapsulek-0058250, a webpage archived on 26.05.2022) by indicating that 1 tablet comprises: a GlauProtect formula (a green tea leaf extract comprising 50 % ECGC, a gingko biloba leaf extract EGb761, a Japanese pagoda tree fruit extract comprising 98 % of genistein) 334 mg, zinc 10 mg, selenium 55 µg.

Moreover, as indicated above, the known GlauCaps V1 formulation contains titanium dioxide, and in accordance with the Commission Regulation (EU) 2022/63 of January 14, 2022, any food containing titanium dioxide (E 171) placed on the market by the manufacturer by August 7, 2022 may remain on the market only until the best-before date or the minimum durability date. Therefore, food, including dietary supplements containing titanium dioxide (E 171), may remain on sale only while the stocks last.

Additionally, it is worth noting that the GlauCaps V1 formulation contains pork gelatin, that limits its use for vegans. In medicinal formulations, pork gelatin is often the main component of the capsule shell. However, it is limited in use by people who exclude all animal products from their diet.

Therefore, there is a need to develop a pharmaceutical formulation that, at specific doses of genistein, would expand the possibilities of non-surgical treatment of glaucoma and/or ocular hypertension, and thus solve an existing technical problem in the field of medicine, in particular in ophthalmology.

The object of the invention is thus to provide a pharmaceutical formulation comprising genistein, which assists patients (i.e. humans and animals) being treated for ocular hypertension and/or glaucoma, as well as being in a group at risk of developing these diseases, while simultaneously reducing the pathological influence of transforming growth factor beta (TGF-β in short) on trabecular meshwork cells and lamina cribrosa of the sclera cells and supporting a neuroprotection process of retinal ganglion cells.

More specifically, the object of the invention is to provide a pharmaceutical formulation comprising genistein enabling higher concentrations of genistein to be obtained in the aqueous humor of the eyeballs and in blood serum in patients treated because of ocular hypertension and/or glaucoma which evoke a better therapeutic effect after oral supplementation with the pharmaceutical formulation according to the invention compared to the "GlauCaps V1" formulation available on the market.

### Disclosure of the invention

According to the present invention, a pharmaceutical formulation for use in prevention or treatment of ocular hypertension and/or glaucoma in a patient, comprises a pharmaceutically acceptable carrier, diluent, excipient and/or adjuvant, characterized in that it comprises genistein in an amount of 10 to 15 % by weight based on the total weight of the pharmaceutical formulation.

Preferably, the pharmaceutical formulation for use according to the invention comprises 12.9 % by weight of genistein, based on the total weight of the pharmaceutical formulation.

Preferably, the pharmaceutical formulation for use according to the invention comes in the form of a capsule with a hydroxypropylmethylcellulose (HPMC) shell.

Preferably, the pharmaceutical formulation for use according to the invention further comprises at least one pharmaceutically acceptable adjuvant.

Preferably, the pharmaceutical formulation for use according to the invention comprises at least one of adjuvant selected from the group consisting of a dry ethanol-water extract from gingko biloba leaves comprising at least 24 % of flavonoid glycosides and at least 6 % of terpene lactones (EGb761), epigallocatechin gallate (EGCG), crocin and/or ashwaghanda.

Preferably, the pharmaceutical formulation for use according to the invention is to be administered in a daily dose including 55 to 85 mg of a daily dose of genistein.

### Beneficial effects and advantages of the invention

According to the present invention, administering to patients a pharmaceutical formulation comprising genistein causes a decrease in intraocular pressure in patients both treated and not treated pharmacologically concomitantly. The present invention makes therefore a novel solution to the above problem concerning non-surgical treatment of glaucoma by pharmacologically reducing production of aqueous humor or facilitating its outflow from the eyeball. Thanks to this invention, a therapeutic effect is achieved in the form of a decrease in intraocular pressure.

The present invention thus allows to meet the existing demand for non-surgical treatment of glaucoma and/or ocular hypertension by providing a pharmaceutical formulation comprising genistein, at specific doses of genistein, for use in prevention or treatment of glaucoma and/or ocular hypertension having a neuroprotective, antioxidant, intraocular pressure-decreasing effects, improving microcirculation in the structures of the eye and increasing cell nutrition.

At the base of the present invention lie the results of research conducted by the present inventors, that allowed to determine the impact *in vivo* in patients treated for glaucoma and/or ocular hypertension.

Glaucoma is a progressive optic neuropathy associated with a change in the phenotype of the lamina cribrosa of the sclera and a secondary process to this of accelerated apoptosis of retinal ganglion cells as a result of neurotrophic deprivation. The fundamental role in the pathomechanism of glaucomatous neuropathy is played by transforming growth factor beta (TGF-β), a cytokine that biochemically determines the process of pathological reconstruction of the extracellular matrix of trabecular meshwork (TM) structures and the optic nerve disc.

### Effect on trabeculation of the iridocomeal angle

The state of tension of TM cells, the density of the extracellular matrix (ECM) and the susceptibility of the filtration angle structures regulate the outflow of aqueous humor (AH), and thus determine the intraocular pressure (IOP) level. TGF-β has the ability to influence the number and phenotype of TM cells, leading to increased resistance within the iridocomeal angle structures and a secondary increase of IOP. TGF-β1, by increasing the expression and production of α-smooth muscle actin (α SMA), induces a "myofibroblast-like" phenotype in TM cells. TM cells altered in this way, comprising highly organized actin filaments, called stress fibers, show functional features of smooth muscle cells (SMLC). Due to their ability to contract and the connection of their cytoskeleton to the ECM, SMLCs actively influence the level of aqueous humor outflow at the trabecular meshwork level. TGF-β1 directly stimulates SLMC contraction. TGF-β1 decrease the matrix GLA protein (MGP) expression, by increasing TM stiffness and reducing the efficiency of the trabecular meshwork aqueous humor pump. TGF-β2 reduces the population of TM cells, increasing their apoptosis and inhibiting proliferation. TGF-β2 induces the secretory phenotype of TM cells, leading to an increase in its trabecular meshwork hydrostatic resistance by increasing collagen synthesis within the ECM.

### Effect on the lamina cribrosa of the sclera

TGF-β plays a key role in the pathognomonic for glaucomatous neuropathy change in the phenotype of the lamina cribrosa of the sclera (LCS). In the structure of the eyeball, the lamina cribrosa of the sclera cells and of the trabecular meshwork cells are the cells with the most similar phenotypes and genotypes to each other. Their genotypic compatibility reaches over 97 % (Steely T.H., 2000). Similarities also extend to the process of their embryogenesis - both cell populations originate from the neural crest and are formed from the 12th week of fetal life. TGF-β1 induces the transformation of LCS cells (analogous as TM) towards smooth muscle cell-like cells (SMLC). Such phenotypically altered LCS cells play a fundamental role in the process of pathological remodeling of the structures of the lamina cribrosa of the sclera, which is the basic pathomechanism of secondary neurotrophic deprivation of retinal ganglion cells and the process of their accelerated apoptosis leading to glaucomatous neuropathy.

Genistein from the formulation according to the invention at an experimentally confirmed and clinically significant level, inhibits the pathological impact TGF-β on trabecular meshwork cells and lamina cribrosa of the sclera cells. Genistein from the formulation according to the invention, by protective effect on trabecular meshwork cells and lamina cribrosa of the sclera cells, supports the process of neuroprotection of retinal ganglion cells during treatment of ocular hypertension and glaucomatous neuropathy.

Thanks to the present invention, in patients with glaucoma a concentration of genistein in aqueous humor of the eyes at the level of about 13.6 mg/L was obtained, indicating increased clinical effectiveness of the formulation, whereas the prior art pharmaceutical formulation allowed for obtaining the concentration of genistein in aqueous humor of the eyes at the level of about 2 mg/L only. According to literature data this is a significant achievement, because the therapeutic effect is noticeable at concentrations of genistein in aqueous humor of the eyes above 2 mg/L, to obtain its maximum at the range of 10-15 mg/L (Source: Role of Protein Tyrosine Kinase on Regulation of Trabecular Meshwork and Ciliary Muscle Contractility. M. Wiederholt, J. Groth, O. Strauss Invest Ophthalmol Vis Sci. 1998; 39:1012-1020). Increased concentrations of genistein in venous blood serum and aqueous humor of the eyes of patients with POAG was affected by the use of a hydroxypropylmethylcellulose shell in a V2 pharmaceutical formulation according to the invention as compared with capsules with a gelatin shell of the known GlauCaps V1 formulation, which resulted in increased clinical effectiveness of V2 formulation according to the invention.

Thanks to the present invention, a therapeutic effect is achieved in a form of inhibiting myofibrocytic transformation of trabecular meshwork cells and lamina cribrosa of the sclera, decreasing intraocular pressure in the eye, that supports non-surgical treatment of ocular hypertension and glaucoma, and administering genistein to patients causes a decrease in intraocular pressure in patients both treated and not treated pharmacologically concomitantly, according to the following studies.

### Short description of the drawings

The subject-matter of the invention is illustrated in the following embodiments, with reference to the attached drawing, in which:
- **Fig. 1**: Shape change (contraction) of trabecular meshwork cells and lamina cribrosa of the sclera caused by increase of SMA under the influence of increasing concentration of TGFβ caused by the activity of increased hydrostatic pressure.
- **Fig. 2**: Influence of genistein on relaxation of trabecular meshwork structures, Fig. 2a and Fig. 2b.
- **Fig. 3**: Concentration of genistein in blood serum for V1/V2/Placebo study groups.
- **Fig. 4**: Concentration of genistein in aqueous humor of the eyeball for V1/V2/Placebo study groups.
- **Fig. 5**: Assessment of the influence of genistein on viability of human trabecular meshwork cells (HTMC).
- **Fig. 6**: Assessment of the level of SMA mRNA after stimulation of the myofibroblastic transformation process of HTMCs using TGFβ and simultaneous blocking of the process by various concentrations of genistein.
- **Fig. 7**: Assessment of the level of α-SMA protein after stimulation of the myofibroblastic transformation process of HTMCs using TGFβ and simultaneous blocking of the process by various concentrations of genistein.
- **Fig. 8**: Visualization of αSMA protein expression changes by means of the immunofluorescence technique - after stimulation of the myofibroblastic transformation process of HTMCs using TGFβ and simultaneous blocking of the process by various concentrations of genistein Fig. 8a) 1, 2, 3, 4 mg/L; Fig. 8b) 5, 10, 15 and 20 mg/L.
- **Fig. 9**: Intraocular pressure before and after use of the V2 pharmaceutical formulation according to the invention - 12-month observation. R - right eye, L - left eye.
- **Fig. 10**: Modification of 24 hour biorhythm of changes in the volume of the eyeball after use of the V2 pharmaceutical formulation according to the invention; Light color curve - an original profile, without pharmacotherapy; Dark color curve - a profile after 3 months of monotherapy with the V2 pharmaceutical formulation according to the invention; Fig. 10a) patient 1, Fig. 10b) patient 2.
- **Fig. 11**: Influence of the V2 pharmaceutical formulation according to the invention on corneal hysteresis and a corneal resistance factor (CRF); Fig. 11a); Increase in corneal hysteresis after use of the V2 pharmaceutical formulation according to the invention, Fig. 11b); Reduction of a corneal resistance factor after use of the V2 pharmaceutical formulation according to the invention.
- **Fig. 12**: Influence of the V2 pharmaceutical formulation of the invention on autoregulation capacity of eye vessels (AC) and an eye critical point (CP); Fig. 12a); Increase in autoregulation capacity of eye vessels (AC) after use of the V2 pharmaceutical formulation according to the invention; Fig. 12b); Increase in an eye critical point (CP) level after use of the V2 pharmaceutical formulation according to the invention.
- **Fig. 13**: Reduction in the ocular pulse amplitude after use of the V2 pharmaceutical formulation according to the invention.

### Embodiments of the invention

The invention is illustrated by the following embodiments. The present invention is not limited only to the detailed embodiments described herein.

### Example 1.

According to the invention, the pharmaceutical formulation according to the invention (herein V2), has the following composition:

| Ingredient | Amount |
|---|---|
| Dry ethanol-water extract from Japanese pagoda tree fruit comprising at least 98 % of genistein | 13.16 mg* |
| Dry ethanol-water extract from green tea leaves comprising at least 50 % EGCG (epigallocatechin gallate) | 33.33 mg |
| Dry ethanol-water extract from ginkgo biloba leaves comprising at least 24 % of flavonoid glycosides and at least 6 % of terpene lactones, EGb 761 | 14.63 mg |
| Zinc oxide | 2.3 mg |
| Sodium selenite | 0.02 mg |
| Microcrystalline cellulose | 18.29 mg |
| Magnesium salts of fatty acids | 0.36 mg |
| Silicon dioxide | 0.36 mg |
| Hydroxypropylmethylcellulose | 17.44 mg |
| Copper complexes of chlorophylls and chlorophyllins | 0.1 1 mg |
| | Total 100 mg |
| *formulation comprises 12.90 mg of pure genistein | |

According to the invention, capsules are prepared from the above ingredients with a total weight of 546.69 mg.

The pharmaceutical formulation according to the invention is obtained in the form of an HPMC capsule by a known encapsulation method. The pharmaceutical formulation for use according to the invention is obtained in the form of a daily dose including from 55 to 85 mg of the daily dose of genistein, or a divided dose.

According to the invention, the pharmaceutical formulation may comprise pharmaceutically acceptable additives, which are pharmaceutically acceptable excipients, carriers, adjuvants, dyes, flavoring substances generally known to those skilled in the art, such as, e.g., silicon dioxide, copper complexes of chlorophylls and chlorophyllins, magnesium salts of fatty acids, microcrystalline cellulose, sodium selenite, zinc oxide, etc., and active and antioxidant adjunctive substances, e.g. crocin, ashwaghanda, EGCG, EGb 761 and others.

### Example 2

Tests concerning the effectiveness of the developed pharmaceutical formulation comprising genistein were carried out with new patients [humans], suffering from normal tension glaucoma or ocular hypertension in order to improve the effect of therapy and prevent disease progression.

According to the invention, the observations show that the obtained results confirmed the beneficial effect of genistein on slowing down the progression of glaucomatous neuropathy and ocular hypertension in patients after administration of the pharmaceutical formulation according to the invention.

This example of the invention focuses on a formulation containing genistein (especially obtained from the Japanese pagoda tree fruit), the use of which in treatment of ocular hypertension and glaucoma has an effect which is:
a) neuroprotective (protecting nerve fibers) towards the optic nerve according to the mechanisms described in the literature, including: inhibiting remodeling of the lamina cribrosa of the sclera, stimulating mitochondrial metabolism - ensuring increased survival of nerve fibers, increasing the amount of the BDNF factor - responsible for the survival of existing neurons as well as promoting the formation of new neurons,
b) antioxidative - increasing the resistance of eye retinal cells to oxidative stress such as e.g. increased pressure or decreased tissue blood supply,
c) decreasing intraocular pressure
d) improving microcirculation in the eye structures, increasing cell nutrition.

According to the invention, *in vivo* observations were carried out on patients treated for glaucoma and/or ocular hypertension.

The aim of the study was to assess the level of genistein presence in the blood serum and aqueous humor of patients who were administered for the subsequent 30 days with 1 capsule of the known GlauCaps^{™} (GlauCaps V1) formulation (OphthaPharm Sp. z o.o., PL) comprising genistein in an amount of 80 mg/capsule of gelatin, and the V2 formulation according to the invention in an amount of 80 mg/capsule of HPMC.

Unexpectedly, an increase in the concentration of genistein in the venous blood serum and aqueous humor of the eyes of patients with POAG (i.e. open-angle glaucoma) was demonstrated by the use of a hydroxypropylmethylcellulose shell in the V2 formulation according to the invention compared to capsules with a gelatin shell of the known GlauCaps V1 formulation, which resulted in an increase of clinical effectiveness of the V2 formulation according to the invention as compared to the known GlauCaps V1 formulation.

### INTRODUCTION:

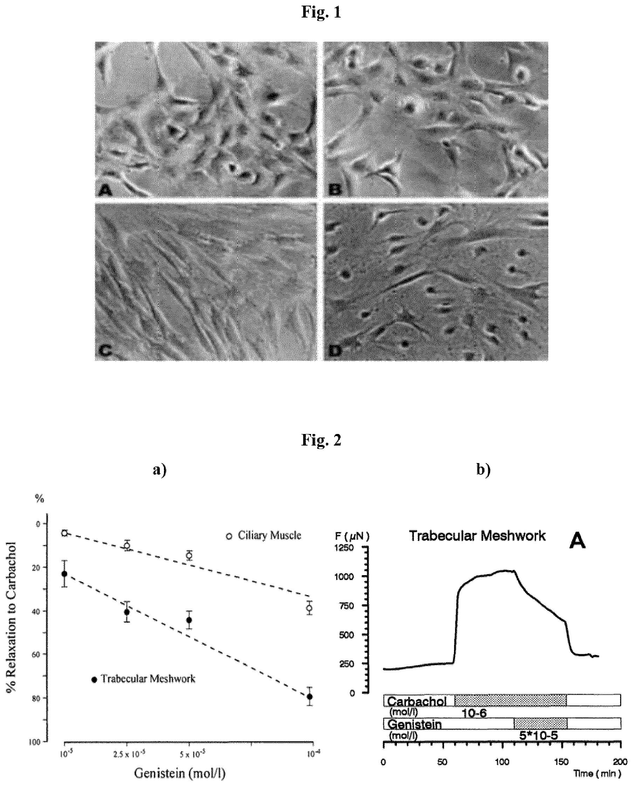

1.1. Glaucoma is a group of progressive optic neuropathies associated with the accelerated process of apoptosis, i.e. the programmed death of retinal ganglion cells as a result of neurotrophic deprivation (Band L.R., 2009; Balaratnasingam C., 2008; Fechtner R.D., Weinreb R.N., 1994; Garcia-Valenzuela E. et al., 1995; Quigley H.A., 1976, 1995, 2000; Yablonski M., Asamoto A., 1993) and pathognomonic changes in the phenotype of the lamina cribrosa of the sclera (Ernest J.T. and Potts A.M., 1968; Quigley H.A. et al., 1983; Roberts M.D. et al., 2009).
1.2. The main biochemical marker of the pathomechanism of glaucomatous neuropathy is TGFβ (transforming growth factor β) - a multifunctional cytokine belonging to the transforming growth factor superfamily. It is present in a significantly higher than original concentration in the aqueous humor of eyes with glaucomatous neuropathy (397 pg/mL vs. 233 pg/mL). The activity of this factor leads to the process of myofibroblastic transformation of trabecular meshwork cells, lamina cribrosa of the sclera and vascular endothelium. It is characterized by the activation of the secretory properties of the above-mentioned groups of cells leading to an increase in the amount of extracellular matrix (E. Lütjen-Drecoll), and an increase in their contractility due to an increase in the amount of α-SMA (M. Wiederholt); see Fig. 1 - Shape change (contraction) of trabecular meshwork cells and lamina cribrosa of the sclera caused by increased SMA under the influence of increasing concentration of TGFβ caused by increased hydrostatic pressure. (Source: Responses of different cell lines from ocular tissues to elevated hydrostatic pressure. Martin B Wax, Gülgün Tezel, Shigeki Kobayashi, M Rosario Hernandez, Br J Ophthalmol. 2000; 84:423-428). The above processes lead to the three key pathomechanisms leading to the development of glaucomatous neuropathy: increased resistance in the aqueous humor outflow tracts, remodeling of the lamina cribrosa of the sclera and dysregulation of blood vessels.
1.3. Genistein - is an organic chemical compound from the group of flavonoids (isoflavones) / phytoestrogens, an agonist of estrogen receptors (ER) and a protein tyrosine kinase (PTK) inhibitor. The biochemical action of genistein is based on the RhoA/ROCK pathway - determining the contractile properties of actomyosin and the permeability of the vascular wall. Genistein at concentrations above 10⁻⁵ mol/L affects the relaxation of trabecular meshwork structures due to the inhibition of pathomechanisms related to excessive activation of TGFβ. See Fig. 2a, below. Clinically significant concentration of genistein determining the relaxation of the above-mentioned structures in approximately 50 % is approximately 2.5-5 × 10⁻⁵ mol/L (6.75-13.5 mg/L). See Fig. 2a and Fig. 2b, (Source: Role of Protein Tyrosine Kinase on Regulation of Trabecular Meshwork and Ciliary Muscle Contractility. M. Wiederholt, J. Groth, O. Strauss. Invest Ophthalmol Vis Sci. 1998; 39:1012- 1020).
2. Assessment of genistein concentration in venous blood serum and aqueous humor of the eyes of patients with POAG (i.e. open-angle glaucoma), after oral supplementation with the known Glaucaps V1 formulation and the V2 pharmaceutical formulation according to the invention from Example 1 of this specification.

### 2.1. Material and methodology

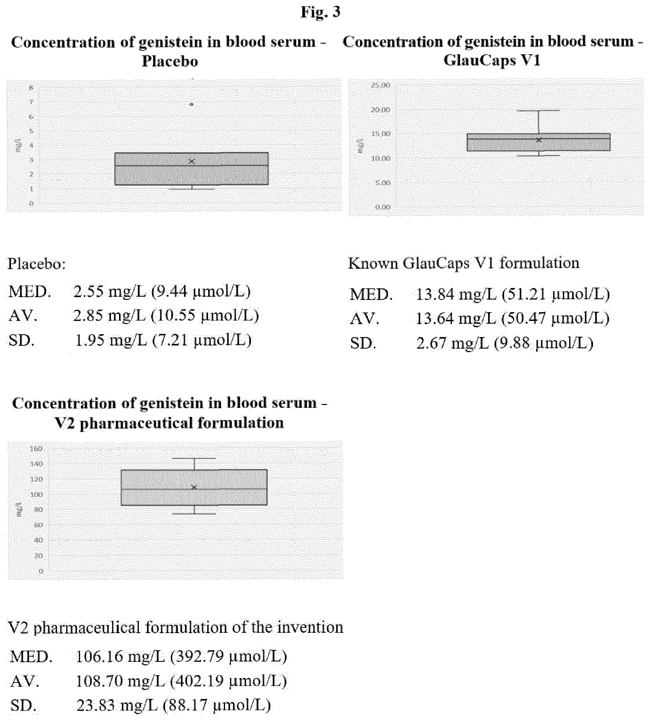

The study groups (V1, V2) and a control group consisted (each) of 12 eyes, 12 patients with POAG for whom cataract surgery was planned as part of the treatment regimen. Each group consisted of 6 men and 6 women, the average age in the groups was 65.4±4.2 years, 64.7±6.1 years and 64.2±4.3 years, respectively. Supplementation with the known GlauCaps V1 formulation/V2 pharmaceutical formulation according to the invention or Placebo (V1/V2 or Placebo - empty HPMC capsule) was started (daily 1 capsule in the evening) 30 days before the planned procedure. The GlauCaps V1 formulation - gelatin capsule shell, the V2 pharmaceutical formulation according to the invention - hydroxypropylmethylcellulose (HPMC) capsule shell. See Fig. 3 - Genistein concentration in blood serum for V1/V2/Placebo study groups.

### 2.2. Biopsy of venous blood and aqueous humor:

Venous blood was collected in the conditions of operating theater immediately before the start of the cataract surgery procedure. Paracentesis with the collection of 0.1-0.2 ml of AH (i.e. aqueous humor) using a needle having 32G diameter through the transparent cornea was performed as part of the cataract surgery procedure.

### Assessment of a genistein concentration:

### 2.3. Measurement of a genistein concentration in venous blood and aqueous humor of the eyeball

To measure a genistein concentration in the above-mentioned body fluids, the liquid chromatography method was employed using H-class Waters Aquity UPLC with a PDA detector.

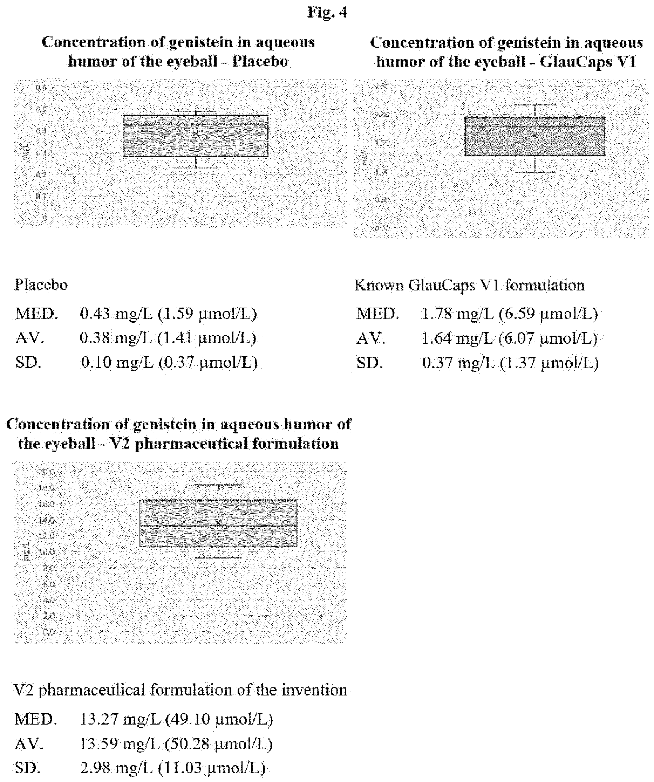

### 2.4. Genistein concentration in the venous blood serum (see Fig. 3) and in the aqueous humor of the eyeball (see Fig. 4).

### 2.5. The ratio of the concentration (CR) of genistein in the blood serum (S) to the concentration of genistein in the aqueous humor (AH) of the eye

| | |
|---|---|
| CR Genistein S/AH | 2.85 / 0.38 = 13.3 % |
| CR GlauCaps V1 Genistein S/AH | 13.64 / 1.64 = 8.3 % |
| CR V2 pharmaceutical formulation Genistein S/AH | 108.70 / 13.59 = 12.5 % |

### 2.6. Conclusions

Oral supplementation with the known GlauCaps V1 formulation and the V2 pharmaceutical formulation according to the invention results in obtaining therapeutically significant concentrations of genistein in the aqueous humor of the eyeballs and blood serum of patients with POAG.

The use of a hydroxypropylmethylcellulose shell (the V2 pharmaceutical formulation according to the invention) significantly increases the concentration of genistein in the venous blood serum and aqueous humor of the eye compared to capsules with a gelatin shell (the known GlauCaps V1 formulation), significantly increasing the clinical effectiveness of the formulation.

### 3. Assessment of the effect of genistein on the viability of human trabecular meshwork cells (HTMC) and inhibition of TGF-β1-stimulated myofibroblastic transformation of HTMCs (expression and production of α-smooth muscle actin (α-SMA))

3.1. Material and methodology: Human trabecular meshwork cells (HTMC - ScienCell Research Laboratories Carlsbad, CA, USA) were supplemented with 2 % fetal bovine serum (FBS), 1 % trabecular meshwork cell growth supplement and 1 % penicillin/streptomycin solution and incubated at 37°C in a humidified atmosphere (5 % CO₂, 95 % O₂) to a state of subfluidity. HTMCs were washed with PBS, and the medium was changed to an experimental medium (EM) in which FBS was replaced with 0.2 % BSA. Then, HTMCs were incubated with the experimental medium (EM) for 24 h, and then the cells from the study group were treated with genistein for 24 h at the following concentrations: 1, 2, 3, 4, 5, 10, 15, 20 mg/L.

### 3.2. Assessment of viability (metabolic activity) of HTMCs

Methodology: HTMCs prepared as described (section 3.1) were treated with a MTT solution (phenyl-tetrazolium bromide - 0.5 mg/mL) for 1 h, then the medium was removed, and the purple formazan crystals were dissolved in 100 µl of DMSO. The optical density of the samples was measured using a Synergy 2 microplate reader (BioTek Instruments, Winooski, VT, USA).

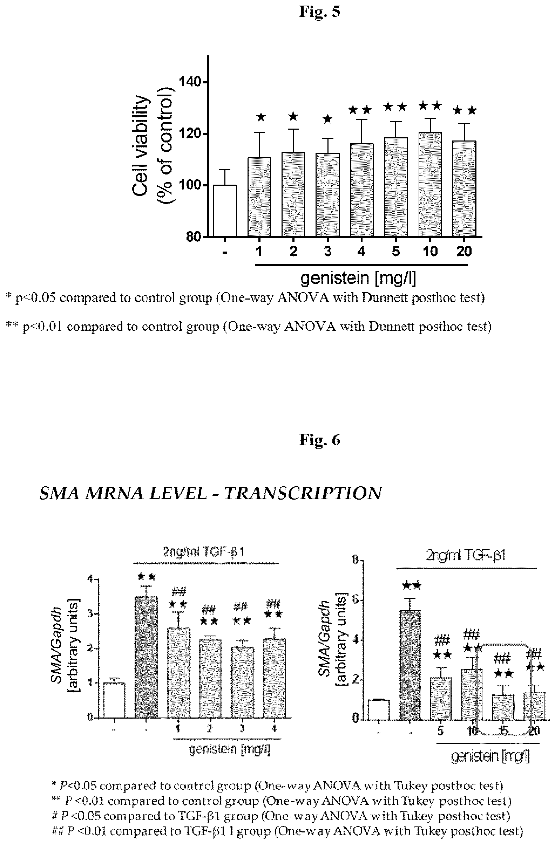

3.2.1. Use of genistein increased the viability of HTMCs. The increase in genistein concentration resulted in the proportional increase in HTMC viability from 10 % at a concentration of 1 mg/L to 20 % at a concentration of 10 mg/L and higher. See Fig. 5 - HTMC viability depending on genistein concentration.

### 3.3. Assessment of the level of inhibition of myofibroblastic transformation of HTMCs

Methodology: HTMCs prepared as described (section 3.1) were stimulated with TGF-1β (2 ng/mL) for the subsequent 24 hours. Semi-quantitative assessment of mRNA and α-SMA protein expression levels was performed using Real-Time PCR and Western blot techniques. The immunofluorescence technique was used to visualize changes in the α-SMA protein expression.

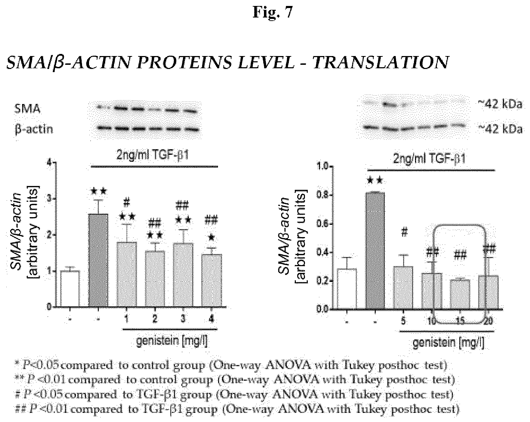

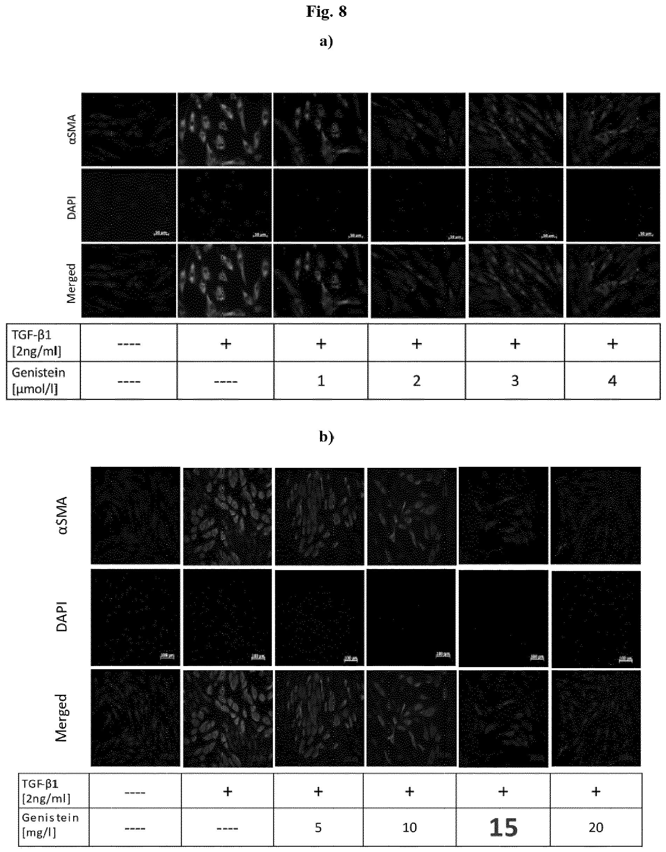

3.3.1. Use of genistein reduced the level of myofibroblastic transformation of HTMCs assessed by the level of mRNA and SMA expression. An increase in the genistein concentration resulted in a proportional reduction in the myofibroblastic transformation of HTMCs at the transcription level (mRNA assessment) from 40 % for 1 mg/L to 100 % for concentrations of 15 mg/L and higher. See Fig. 6 - Assessment of the level of SMA mRNA after stimulation of the myofibroblastic transformation process of HTMCs using TGFβ and simultaneous blocking of this process by various concentrations of genistein. At the most clinically important translation level (α-SMA protein assessment), the reduction in transformation was 40 % for 1 mg/L to 120 % for concentrations of 10 mg/L and higher. See Fig. 7 - Assessment of the level of α-SMA protein after stimulation of the myofibroblastic transformation process of HTMCs using TGFβ and simultaneous blocking of this process by various concentrations of genistein. The immunofluorescence technique was used to visualize changes in the phenotype and αSMA protein expression level of HTMCs. Cells treated with TGFβ and low concentrations of genistein show a rounded shape in their morphology and a higher level of fluorescence (resulting from the increased presence of intracellular contractile elements - SMA). Cells exposed to TGFβ and high concentrations of genistein show an elongated shape in their morphology and a lower level of fluorescence (resulting from a reduced or normalized level of intracellular contractile elements - SMA). See Fig. 8 - Visualization of changes in the αSMA protein expression by means of the immunofluorescence technique - after stimulation of the myofibroblastic transformation process of THTMC using TGFβ and simultaneous blocking of the process by various concentrations of genistein (Fig. 8a - genistein concentrations equivalent to those achieved in the aqueous humor of the eyeball during oral administration the known GlauCaps V1 formulation; Fig. 8b - genistein concentrations equivalent to those achieved in the aqueous humor of the eyeball during oral use of the V2 pharmaceutical formulation according to the invention).

### 3.4. Conclusions

Genistein in concentrations equivalent to those achieved in the aqueous humor of the eyeball during oral use of the V2 pharmaceutical formulation according to the invention (13.6 ± 3 mg/L) causes
A. 20 % increase in viability and
B. 4-fold reduction in TGF-β1-stimulated SMA mRNA expression and α-smooth muscle actin (α SMA) production in HTMC cells (inhibition of myofibroblastic transformation)

### 4. Clinical implications

4.1. The V2 pharmaceutical formulation according to the invention increases the viability and inhibits the pathological effect of TGF-β1 on HTMCs, conditioning homeostasis of the aqueous humor outflow tracts and promoting the baroprotective effect (decrease in intraocular pressure).

Material: 40 people, 22 women, 18 men aged 50 ± 11.7 years.

Diagnosis: Ocular hypertension or borderlines of IOP and positive family history of glaucoma.

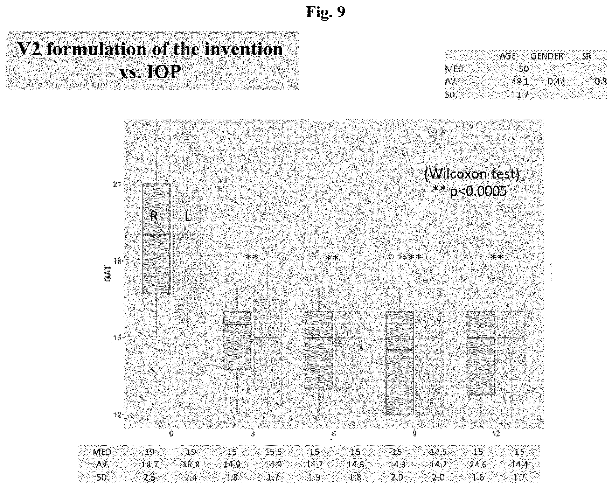

Assessment of the baseline level of the intraocular pressure (IOP) without pharmacotherapy and after 3, 6, 9 and 12 months of monotherapy with the V2 pharmaceutical formulation according to the invention. There is a significant reduction in IOP from an average level of 18.7 mmHg to 14.5 mmHg. The phenomenon of steroid response (SR) occurred in 80 % of the study participants (increase in IOP after topical application, see Fig. 9 - Intraocular pressure before and after the use of the V2 pharmaceutical formulation according to the invention - observation for 12 months. R - right eye, L - left eye.

### 4.2. Modification of the biorhythm of eyeball volume changes.

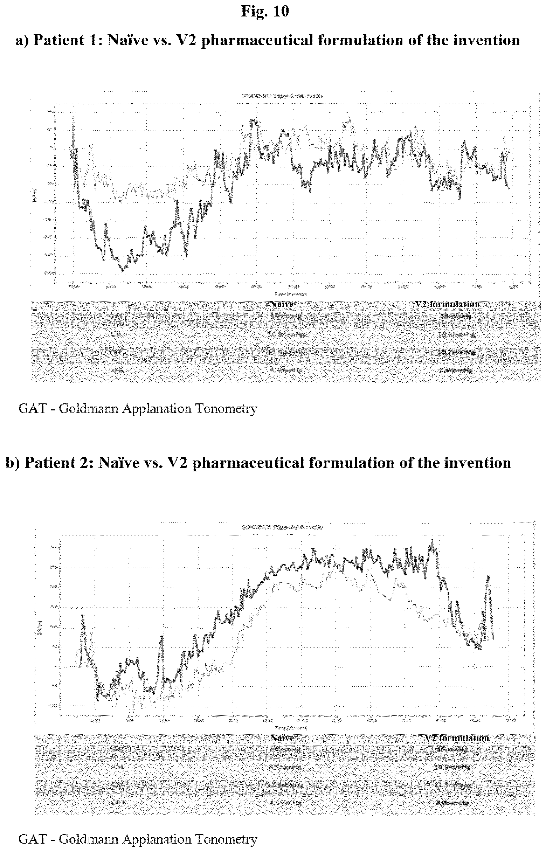

The V2 pharmaceutical formulation according to the invention, by influencing the homeostasis of the aqueous humor outflow tract, has not only a baroprotective effect (decrease in IOP), but also the potential to modify the profile of the biorhythm of changes in the volume of the eyeball and, due to the genotypic and phenotypic identity of HTMCs and lamina cribrosa of the sclera cells, it also has protective effect on the lamina cribrosa of the sclera, supporting the neuroprotection process of retinal ganglion cells. See Fig. 10 a) and Fig. 10 b) - Modification of the 24-hour biorhythm of changes in the volume of the eyeball after the use of the V2 pharmaceutical formulation according to the invention; Light color curve - an original profile, without a pharmacotherapy; Dark color curve - a profile after 3 months of the monotherapy with the V2 pharmaceutical formulation according to the invention; Fig. 10 a) patient 1, Fig. 10 b) patient 2.

### 4.3. Modification of corneal hysteresis and corneal resistance factor after the use of the V2 pharmaceutical formulation according to the invention

Corneal hysteresis (CH) is a biomechanical property that reflects the ability of the cornea to absorb and dissipate energy (mainly its viscous properties). The corneal resistance factor (CRF) is a property that determines the elastic properties of the cornea. Functionally, for the elastic properties of the cornea are responsible collagen fibers thereof, the basic substance composed of glycosaminoglycans and proteoglycans, which provides its viscous properties. Corneal hysteresis is lower (approx. 1-2 mmHg) and it decreases faster in eyes with glaucoma compared to normal eyes. Moreover, CH correlates negatively with structural and functional changes in glaucoma and with the response to baroprotective therapy. Healthy patients have a thicker cornea with a healthy stroma and a more viscous basic substance due to normal GAG and PG (abbreviations: GAG, i.e. Glycosaminoglycans and PG, i.e. Proteoglycans), which results in higher CH. The quantity and quality of corneal viscous substances (GAG and PG) responsible for corneal hysteresis (CH) is reduced with age, resulting in decreased CH. The rate of the CH decline over time is estimated to range from 0.24 to 0.7 mmHg per ten years in healthy eyes. CH is positively correlated with central corneal thickness (CCT) among healthy people (a much weaker positive correlation occurs in patients with glaucoma) and negatively correlated with intraocular pressure (the higher the IOP, the lower the CH). CRF is positively correlated with IOP and CCT and increases with age. The Ocular Response Analyzer is used to measure the biomechanical properties of the cornea and compensated IOP (IOPcc) in vivo.

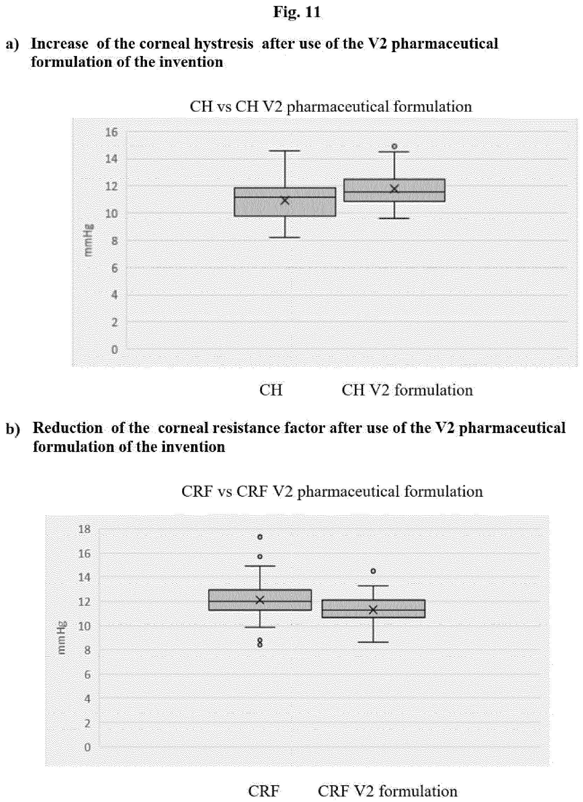

The use of the V2 pharmaceutical formulation according to the invention causes a clinically and statistically significant increase in corneal hysteresis CH and a decrease in CRF, leading to a reduced risk of glaucoma development and progression, see Fig. 11.

The median, mean and standard deviation and their differences before the use of the V2 pharmaceutical formulation according to the invention (IOPcc, CH, CRF) and after the use of the V2 pharmaceutical formulation according to the invention (V2IOPcc, V2CH, V2CRF) are presented in Table 1:

**Table 1.**

| | IOPcc | V2IOPcc | Difference | CH | V2CH | Difference | CRF | V2CRF | Difference |
|---|---|---|---|---|---|---|---|---|---|
| Median | 18.6 | 13.0 | -4.2 | 11.2 | 11.6 | 0.7 | 12.0 | 11.3 | -0.8 |
| Average | 18.4 | 13.9 | -4.6 | 11.0 | 11.8 | 0.8 | 12.1 | 11.3 | -0.8 |
| Standard deviation | 3.2 | 3.0 | 2.2 | 1.4 | 1.2 | 0.7 | 1.7 | 1.3 | 0.8 |

The increase in CH and decrease in CRF after the use of the V2 pharmaceutical formulation according to the invention occurs as a result of the direct effect of the formulation on fibroblastic cells and the basic substance of the corneal stroma, and through its baroprotective activity.

### 4.4. Modification of functional parameters of the eyeball vessels

Functional disorders of the eyeball vessels (dysregulation) are among the main causes of the glaucoma development and progression. Clinically, the two main ones are decreased autoregulatory capacity (changes in a vessel diameter depending on blood pressure) of the eye arteries - Autoregulatory Capacity (AC) of the middle artery and short posterior ciliary arteries, and a decreased Critical Point (CP) of these vessels (a value of the intraocular pressure at which there is a lack of perfusion). In a healthy eye, AC is 27.4 mmHg and CP is 40.1 mmHg. The Ocular Pulse Blood Flow Analyzer (OPFA) is used to measure the functional parameters of the eyeball vessels.

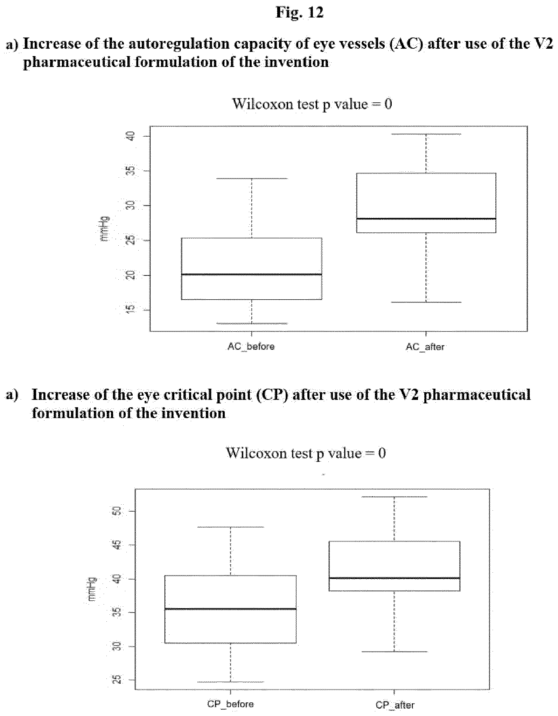

The use of the V2 pharmaceutical formulation according to the invention causes a clinically and statistically significant increase in AC and CP, leading to a reduced risk of glaucoma development and progression, See: Fig. 12a and Fig. 12b.

The increase in AC and CP values after the use of the V2 pharmaceutical formulation according to the invention occurs as a result of the direct relaxing effect of the formulation on endothelial cells and the walls of eye arteries, also leading to the normalization of the Ocular Pulse Amplitude of the eye (see section 4.5).

### 4.5. Modification of the Ocular Pulse Amplitude

Ocular Pulse Amplitude (OPA) describes short-term fluctuations in the volume of the eyeball caused by the influence of the cardiovascular system. Increased pulse amplitude causes excessive mechanical load on the structures of the eyeball wall, leading to a progressive deterioration of its biomechanical properties, resulting in a reduction of corneal hysteresis and an increase in CRF, which increases the risk of glaucoma development and progression. The Pascal Dynamic Contour Tonometer (DCT) is used to measure OPA and DCT pressure.

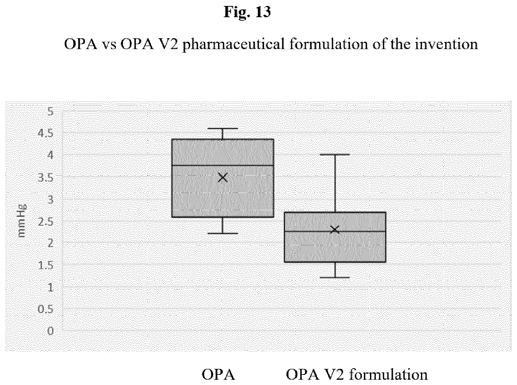

The use of the V2 pharmaceutical formulation according to the invention at a clinically and statistically significant level reduces OPA, reducing stress leading to the remodeling of the eyeball wall, and thus reduces the risk of glaucoma development and progression, see Fig. 13.

The median, average and standard deviation of DCT/OPA and their differences before the use of the V2 pharmaceutical formulation according to the invention (DCT and OPA) and after the use of the V2 pharmaceutical formulation according to the invention (V2DCT and V2OPA) are presented in Table 2:

**Table 2.**

| | DCT | V2DCT | DIFFERENCE | OPA | V2OPA | DIFFERENCE |
|---|---|---|---|---|---|---|
| MEDIAN | 23.4 | 18.4 | 4.7 | 3.8 | 2.3 | 1.1 |
| AVERAGE | 22.8 | 18.1 | 4.7 | 3.5 | 2.3 | 1.2 |
| SD | 2.2 | 2.2 | 2.0 | 0.8 | 0.8 | 0.6 |

The decrease in OPA after the use of the V2 pharmaceutical formulation according to the invention occurs as a result of the direct relaxing effect of the formulation on the endothelial cells/wall of the eyeball vessels and through the baroprotective activity.

## Claims

1. A pharmaceutical formulation for use in prevention or treatment of ocular hypertension and/or glaucoma in a patient, comprising a pharmaceutically acceptable carrier, diluent, excipient and/or adjuvant, **characterized in that** it comprises genistein in an amount of 10 to 15 % by weight based on the total weight of the pharmaceutical formulation.

2. The pharmaceutical formulation for use according to claim 1, **characterized in that** it comprises 12.9 % by weight of genistein, based on the total weight of the pharmaceutical formulation.

3. The pharmaceutical formulation for use according to claim 1 or 2, **characterized in that** the formulation comes in the form of a capsule with a hydroxypropylmethylcellulose (HPMC) shell.

4. The pharmaceutical formulation for use according to claims 1-3, **characterized in that** it further comprises at least one pharmaceutically acceptable adjunctive substance.

5. The pharmaceutical formulation for use according to claim 4, **characterized in that** the adjunctive substance is selected from the group including a dry ethanol-water extract from gingko biloba leaves comprising at least 24 % of flavonoid glycosides and at least 6 % of terpene lactones (EGb761), epigallocatechin gallate (EGCG), crocin and/or ashwaghanda.

6. The pharmaceutical formulation for use according to claim 1-5, **characterized in that** the formulation is administered in a daily dose including 55 to 85 mg of a daily dose of genistein.
